# EUROPÄISCHE PATENTSCHRIFT

(11) **EP 0 882 722 B1**
(45) Veröffentlichungstag und Bekanntmachung des Hinweises auf die Patenterteilung: **27.03.2002**
(21) Anmeldenummer: 98109726.4
(22) Anmeldetag: 28.05.1998
(51) Int. Cl.: C07D 311/72

(54) **Herstellung von alpha-Tocopherol**
Preparation of alpha-tocopherol
Préparation d'alpha-tocophérol

(30) Priorität: 06.06.1997 EP 97109174
(43) Veröffentlichungstag der Anmeldung: 09.12.1998
(73) Patentinhaber: F. HOFFMANN-LA ROCHE AG, 4070 Basel (CH)
(72) Erfinder: Breuninger, Manfred, 79713 Bad Säckingen (DE)

(56) Entgegenhaltungen:
- EP-A- 0 176 690
- EP-A- 0 178 400
- EP-A- 0 338 429
- EP-A- 0 769 497
- DE-A- 2 351 272
- US-A- 2 486 539
- US-A- 2 519 863

## Beschreibung

Die vorliegende Erfindung betrifft ein Verfahren zur Permethylierung von sogenannten "Nicht-α-Tocopherolen" zu α-Tocopherol mit Methanol oder einem äquivalenten gasförmigen Gemisch unter nahe- oder überkritischen Bedingungen sowie unter Verwendung eines besonderen Katalysators.

Bekanntlich unterscheiden sich die natürlich vorkommenden Nicht-α-Tocopherole β-, γ- und δ-Tocopherol von dem die höchste Vitamin E-Aktivität aufweisenden und somit biologisch wertvollsten Tocopherol, α-Tocopherol, durch das Fehlen einer oder zweier Methylgruppen in 5- und/oder 7-Stellung des Chromanteils des Moleküls. Daher besteht ein Bedürfnis, derartige Nicht-α-Tocopherole in das α-Tocopherol chemisch überzuführen, wobei das Hauptproblem an der effizienten, ergänzenden Mono- bzw. Dimethylierung des Benzolrings der substituierten Chromanylgruppe liegt.

Da sich synthetische Verfahren zur Herstellung von naturidentischem α-Tocopherol bislang als unwirtschaftlich erwiesen haben und natürliche, insbesondere pflanzliche, Quellen von Tocopherolen in der Regel neben einem relativ geringen Anteil an α-Tocopherol überwiegend Nicht-α-Tocopherole enthalten, weshalb auch die Isolierung des α-Tocopherols aus solchen natürlichen Materialien (Rohmaterialien) unrentabel ist, besteht die Aufgabe der vorliegenden Erfindung darin, ein Verfahren zur Umwandlung von, eventuell in einschlägigen Rohmaterialien, vorhandenen oder aus diesen gewonnenen Nicht-α-Tocopherolen in α-Toco-pherol zur Verfügung zu stellen, welches in vielerlei Hinsicht wirtschaftlicher ist als die bisherigen einschlägigen Verfahren.

Im Sinne dieser Erfindungsaufgabe sind bereits einige Verfahren zur Umwandlung von Nicht-α-Tocopherolen in α-Tocopherol aus dem Stande der Technik bekannt geworden. Beispielsweise offenbart die europäische Patentpublikation (EP) 176 690 (Henkel Corporation) ein Verfahren zur Methylierung von Nicht-α-Tocopherolen durch Einsatz eines Methylierungsmittels in der Gas/Flüssig-Phase und in Gegenwart eines Metall-oxidkatalysators; es handelt sich dabei um eine direkte, einstufige Methylierung des Chromanolrings, die eine vollständige Tocopherol-Methylierung sein soll, also um eine Permethylierung.

Der zu diesem Zweck verwendete Katalysator wird in EP 176 690 "funktionell" definiert im Sinne, dass jeder Katalysator, welcher zur Induktion einer Alkylierungsreaktion befähigt ist, eingesetzt werden kann; er sei allerdings typischerweise ein Metalloxid oder Gemisch mehrerer Metalloxide, dessen bzw. deren Metallatom(e) aus den Gruppen IIA, IIB, IIIA, IVA, IVB, VB, VIB, VIIB und VIII des Periodensystems ausgewählt ist bzw. sind. Als bevorzugte Metalloxide sind die Oxide von Be, Mg, Ca, Ti, Zr, V, Mo, Cr, Mn, Tc, Fe, Co, Ni, Zn, Cd, In, Sn, Si, Al, La, Ce, Pr und Nd angegeben. Solche Katalysatoren können als solche ("neat") oder auf einem inerten Trägermaterial verwendet und auf irgendeine geeignete Weise, sogar in situ, hergestellt werden. Bei der in situ-Herstellungsweise wird beispielsweise ein Metallsalz in den Reaktor eingeführt und anschliessend zum entsprechenden Metalloxid umgesetzt oder zersetzt. Nach Entfernung von Nebenprodukten und allfällig unreagierten Reaktanden kann die Methylierung erfolgen. Gemäss einem Beispiel der "externen" Herstellung eines solchen Katalysators wird trockenes Zinnhydroxid zu einer Lösung von Ammoniumvanadat in wässriger Oxalsäure gegeben und mit einer ebenfalls zugegebenen gealterten Lösung von partiell polymerisiertem Siliziumhydroxid versetzt, wobei ein Niederschlag entsteht. Dieser wird getrocknet, kalziniert und unter Formgebung verpresst. In einem weiteren Beispiel der EP 176 690 wird festes Titandioxid zu einem wässrigen Gemisch von Ammoniumvanadat und Oxalsäure gegeben und das neue Gemisch erhitzt und getrocknet, dann kalziniert und zu Festformen verpresst. In beiden Fällen entstehen Oxidmischungen, die allerdings kristallographisch nicht als Mischoxide bezeichnet werden können; vielmehr sind sie Vanadiumoxid auf einem Zinnoxid/Siliziumdioxid- bzw. Titandioxid-Träger. In der EP 176 690 ist keine Rede von Hydrotalciten oder Hydrotalcitähnlichen Metallhydroxycarbonaten als möglichen Metall-enthaltenden Materialien, aus welchen die Metalloxid- oder Mischoxid-Katalysatoren hergestellt werden könnten, geschweige denn von Kupferoxid enthaltenden Katalysatoren (Kupfer gehört der Gruppe Ib des Periodensystems an).

Ferner wird in der EP 176 690 als besonders günstiger Temperaturbereich derjenige von etwa 390 bis etwa 470°C und als bevorzugter Druck der Umgebungsdruck (Normaldruck) erwähnt. Vorgesehen wird ausserdem die Verwendung von überschüssigem Methylierungsmittel oder einem inerten Trägergas, z.B. Stickstoff, nicht aber von einem (zusätzlichen) Lösungsmittel.

Es wurde nun überraschenderweise gefunden, dass dieses bekannte Verfahren der Henkel Corporation durch eine ganz besondere Wahl des Katalysators und der sonstigen Reaktionsbedingungen entscheidend verbessert werden kann. Gegenstand der vorliegenden Erfindung ist ein Verfahren zur Umwandlung von Nicht-α-Tocopherolen in α-Tocopherol durch katalysierte Permethylierung mindestens eines Nicht-α-Tocopherols mit einem Methylierungsmittel, das dadurch gekennzeichnet ist, dass man als Methylierungsmittel im nahe oder überkritischen Druck- und Temperaturbereich befindliches Methanol oder ein aus Wasserstoff und Kohlenmonoxid und/oder Kohlendioxid bestehendes, dem Methanol äquivalentes Gemisch, und als Katalysator einen aus Hydrotalciten hergestellten Mischoxid-Katalysator, der zumindest Kupfer- und Magnesiumoxid sowie mindestens ein Oxid eines dreiwertigen Metalls aufweist, verwendet, und die Permethylierung bei einem Druck zwischen etwa 50 bar und etwa 120 bar (etwa 5 bis etwa 12 MPa) und bei einer Reaktionstemperatur im Bereich von etwa 240°C bis etwa 350°C durchführt.

Wie bereits oben angedeutet, kann im Prinzip als Edukt im erfindungsgemässen Verfahren ein Rohmaterial, das zumindest ein Nicht-α-Tocopherol, z.B. β-, γ-' oder δ-Tocopherol, enthält, oder ein aus einem solchen Rohmaterial gewonnenes oder anderweitig erhaltenes Tocopherolgemisch, das ebenfalls zumindest ein Nicht-α-Tocopherol enthält, eingesetzt werden. Das Gewinnen oder anderweitiges Erhalten der Tocopherolgemische erfolgt nach längst bekannten Methoden. Da bekanntlich pflanzliche Oele und Fette, wie beispielsweise Sojaöl, Rapsöl, Baumwollsaatöl, Erdnussöl, Weizenkeimöl, Maisöl, Gerstenöl, Roggenöl, Distelöl und dergleichen, wertvolle natürliche Quellen von Tocopherolen (u.a. α- und Nicht-α-Tocopherolen) sind, können solche Oele oder vorzugsweise deren Destillate, Konzentrate und andere Produkte, die einen höheren Gehalt an Tocopherolen aufweisen und weniger unerwünschte andere Bestandteile, z.B. Sterole, freie und veresterte Fettsäuren, Wachse und Glyzeride, enthalten, als Edukt im erfindungsgemässen Verfahren Verwendung finden. Die Anwesenheit von Sterolen und den weiteren genannten Bestandteilen beeinträchtigt das erfindungsgemässe Verfahren allerdings nicht wesentlich. Insbesondere Distelöl und Sojaöl erweisen sich als wertvolle Quellen von Tocopherolen, u.a. α-Tocopherol und den in dieses erfindungsgemäss überzuführenden Nicht-α-Tocopherolen. Es spielt natürlich keine Rolle, dass sich u.a. α-Tocopherol selber im Edukt befindet, da das α-Tocopherol die Ueberführung der Nicht-α-Tocopherole in α-Tocopherol nicht verhindert und selber unreagiert im Verfahrensprodukt bleibt.

Bei den im erfindungsgemässen Verfahren verwendeten Druck- und Temperaturbedingungen handelt es sich um den nahe- oder überkritischen Bereich in dem sich das erhitzte und unter Druck stehende Methanol während der Permethylierung befindet. Methanol, das einen Druck von mindestens etwa 50 bar (5 MPa) und eine Temperatur von mindestens etwa 240° C aufweist, befindet sich nämlich in diesem Bereich. Der überkritische Bereich fängt bei dieser Mindestemperatur mit einem Druck von etwa 77,5 bar (7,75 MPa) an. Diese physikalischen Daten entsprechen gleichzeitig natürlich auch den Drücken resp. Temperaturen, bei denen das erfindungsgemässe Verfahren als Ganzes (effektiv unter "nahe- oder überkritischen Bedingungen"), d.h. die katalysierte Permethylierung, durchgeführt wird. Ein Zusatz von bis zu etwa 20 Volumenprozent Wasser zum Methanol kann die Selektivität der Methylierung steigern, verringert jedoch deren Geschwindigkeit.

Als Alternative zum im nahe- oder überkritischen Bereich befindlichen Methanol kann im erfindungsgemässen Verfahren entweder ein dem Methanol äquivalentes Gemisch aus Wasserstoff und Kohlenmonoxid oder das äquivalente Gemisch aus Wasserstoff und Kohlendioxid verwendet werden. Beim erstgenannten Gemisch handelt es sich zweckmässigerweise um ein solches Gemisch, welches grundsätzlich zur Synthese von Methanol geeignet ist. Somit enthält dieses Gemisch Wasserstoff und Kohlenmonoxid im molaren Verhältnis von etwa 2:1 oder grösser; es wird unter den gleichen Druck- und Temperaturbedingungen wie Methanol verwendet. Das zweitgenannte "äquivalente Gemisch" ist ebenfalls zweckmässigerweise eines, welches sich grundsätzlich zur Synthese von Methanol eignet. In diesem Fall beträgt das Molverhältnis Wasserstoff:Kohlendioxid etwa 3:1 oder grösser, und das Gemisch wird auch unter den gleichen Druck- und Temperaturbedingungen wie Methanol verwendet. Bezüglich der Synthese von Methanol aus solchen "äquivalenten Gemischen" wird beispielsweise auf Catalysis Today, Vol. 11, No. 2, Seiten 173-291, insbesondere Seiten 230-235 (1991), verwiesen.

Besonders geeignet als äquivalentes Gemisch ist eines, das sowohl Kohlenmonoxid als auch Kohlendioxid enthält, und somit als Kombination beider obengenannter Gasgemische angesehen werden kann.

Die sogenannten Hydrotalcite, aus denen der erfindungsgemäss verwendete Mischoxid-Katalysator hergestellt ist, stellen eine bekannte Klasse in der Natur vorkommender, isomorpher Mineralien dar, welche jeweils gemischte Hydroxycarbonate verschiedener Metalle, z.B. Magnesium und Aluminium oder Magnesium und Eisen, sind. "Hydrotalcit" selber hat die chemische Formel Mg₆Al₂(OH)₁₆CO₃•4H₂O, und weitere Mineralien mit einer ähnlichen Struktur kommen in der Natur vor oder sind synthetisiert worden, wie Sjögrenit und Pyroaurit. Die letzteren Mineralien nennt man konventionell Hydrotalcit-ähnliche Verbindungen; im Rahmen der vorliegenden Erfindung sind Hydrotalcit und Hydrotalcit-ähnliche Verbindungen - der anerkannten allgemeinen Formel [M(II)₁₋ₓM(III)ₓ(OH)₂]^{x+}(Aⁿ⁻x/n).mH₂O, worin M(II) und M(III) zwei- bzw. dreiwertige Metallionen, An- ein austauschbares Anion und x 0,1-0,33 bedeuten, - alle unter dem Sammelbegriff "Hydrotalcite" zu verstehen. Für einschlägige Literatur betreffend Hydrotalcite und deren Herstellung, vor allem durch Kopräzipitation, und Verwendung, zum Teil als solche oder nach Kalzinieren zu gemischten Metalloxiden, als Katalysatoren wird unter anderem auf Catalysis Today, Vol. 11, No. 2, 173-291 (1991) und die darin zitierten Referenzen, Appl. Catalysis A: General 119,241-252(1994), und ibid., 145, 141-153 und 225-230(1996), verwiesen.

Der erfindungsgemäss verwendete, aus Hydrotalciten hergestellte Mischoxid-Katalysator enthält zumindest Kupferoxid und Magnesiumoxid (sowohl Kupfer wie auch Magnesium sind zweiwertige Metalle) sowie mindestens ein Oxid eines dreiwertigen Metalls [nachfolgend als "Metall(III)oxid" bezeichnet]. Bei den diesbezüglichen Metall(III)oxiden handelt es sich vorzugswese um Oxide von Aluminium und Eisen(III). Allerdings können auch Oxide weiterer zweiwertiger und/oder dreiwertiger Metalle vorhanden sein. Beispiele der weiteren zweiwertigen(II) und drei-wertigen(III) Metalle sind Beryllium(II), Calcium(II), Vanadium(III), Chrom(III), Mangan(II), Eisen(II), Cobalt(II), Nickel(II), Zink(II), Gallium(III) und Cadmium(II). Bevorzugt enthält der Mischoxid-Katalysator neben Kupferoxid und Magnesiumoxid nur noch Aluminiumoxid und Eisen(III)oxid.

In dem erfindungsgemäss verwendeten, aus Hydrotalciten hergestellten Mischoxid-Katalysator beträgt das atomare Verhältnis der (gesamten) zweiwertigen Metalle zu dem dreiwertigen Metall oder - falls mehrere Metall(III)oxide vorhanden sind - zu den gesamten dreiwertigen Metallen zweckmässigerweise etwa 2:1 bis etwa 10:1, vorzugsweise etwa 3:1 bis etwa 4:1. Was das atomare Verhältnis zwischen den vorhandenen zweiwertigen Metallen (u.a. zwingend Kupfer und Magnesium) unter sich anbelangt, so beträgt das atomare Verhältnis von Kupfer zu Magnesium bzw. von Kupfer zu Magnesium und weiteren vorhandenen zweiwertigen Metallen zweckmässigerweise etwa 5:95 bis etwa 60:40, vorzugsweise etwa 25:75 bis etwa 50:50. Das atomare Verhältnis zwischen dem vorzugsweise vorhandenen Aluminium und dem ebenfalls vorzugsweise vorhandenen Eisen oder der Gesamtheit der weiteren dreiwertigen Metalle [Al: sonstige Metalle(III)] beträgt vorzugsweise etwa 2:1 bis etwa 1:2, ganz bevorzugt etwa 2:1.

Neben den zwingend vorhandenen Kupferoxid, Magnesiumoxid und Metall(III)oxid(en) können u.a. Oxide von Lithium, Natrium und/oder Kalium im erfindungsgemäss verwendeten Mischoxid-Katalysator vorhanden sein, und zwar in einer Menge, die zweckmässigerweise bis zu etwa 2% des Gesamtgewichts des Mischoxid-Katalysators ausmacht. Diese Alkalimetalle sind normalerweise deswegen vorhanden, weil Alkalimetallbasen zur Herstellung bzw. Aufarbeitung des Mischoxid-Katalysators benötigt und nicht vollständig ausgewaschen werden.

Im Prinzip wird das erfindungsgemässe Verfahren derart durchgeführt, dass man das Nicht-α-Tocopherol, das Gemisch mehrerer Nicht-α-Tocopherole oder das zumindest ein Nicht-α-Tocopherol enthaltende Rohmaterial, jeweils eventuell in einem inerten Lösungsmittel gelöst, zusammen mit Methanol oder dem oben näher erläuterten äquivalenten Gemisch unter (in bezug auf Druck und Temperatur) nahe- oder überkritischen Bedingungen durch einen mit dem Mischoxid-Katalysator beladenen Reaktor, z.B. ein mit dem Katalysator beladenes, geheiztes Rohr, leitet. Das aus dem Reaktor ausfliessende Rohprodukt muss dann lediglich von den in der Methylierung entstandenen Gasen, überwiegend Wasserstoff und Kohlenmonoxid, sowie vom Methanolüberschuss und eventuell verwendeten, übrigbleibenden inerten Lösungsmitteln abgetrennt werden, z.B. durch Destillation. Nach der Methylierung und eventuellen Abtrennung von Gasen, Methanolüberschuss, Lösungsmitteln usw. kann das an α-Tocopherol angereicherte Produkt gewünschtenfalls mehrmals dem erfindungsgemässen Verfahren unterworfen werden, um jeweils ein an α-Tocopherol noch stärker angereichertes Produkt zu erhalten, wenn der beabsichtigte Verwendungszweck dies erforderlich macht, d.h. um den benötigten Umsatzgrad zu α-Tocopherol zu erreichen. Der Natur eines katalytischen Verfahrens entsprechend lässt sich der gewünschte Umsatzgrad auch durch eine Verlängerung der Kontaktzeit des Nicht-α-Tocopherols bzw. des dieses enthaltenden Gemisches oder Rohmaterials am Katalysator erreichen, indem die Katalysatormenge im Reaktor erhöht oder die Fliessgeschwindigkeit des eingesetzten Nicht-α-Tocopherols, Gemisches bzw. Rohmaterials im Reaktor erniedrigt wird. Eine wesentliche Zersetzung der Tocopherole tritt während der Umsetzung normalerweise nicht auf.

Als obenerwähntes inertes Lösungsmittel kommt zweckmässigerweise ein nichtpolares organisches Lösungsmittel in Frage, vorzugsweise ein Alkan mittleren Molgewichts, vor allem ein C₅₋₁₀-Alkan, z.B. Pentan, Hexan oder Heptan, oder Gemische davon, z.B. ein Petrolether mit Siedebereich zwischen etwa 40°C und etwa 120°C; ein cyclisches Alkan, z.B. Cyclohexan; oder ein aromatischer Kohlenwasserstoff, z.B. Toluen.

Falls das zu methylierende Nicht-α-Tocopherol, Gemisch mehrerer Nicht-α-Tocopherole bzw. zumindest ein Nicht-α-Tocopherol enthaltende Rohmaterial in einem inerten Lösungsmittel gelöst wird - und so verdünnt wird -, beträgt die Konzentration des Nicht-α-Tocopherols, der Nicht-α-Tocopherole bzw. des Nicht-α-Tocopherol-Anteils im Lösungsmittel zweckmässigerweise etwa 10 g/l bis etwa 500 g/l, vorzugsweise etwa 100 g/l bis etwa 500 g/l.

Die Menge des als Methylierungsmittel verwendeten Methanols gegenüber dem oder den Nicht-α-Tocopherol(en) entspricht im allgemeinen mindestens einem Aequivalent der (geschätzten) methylierbaren Stellen in dem Nicht-α-Tocopherol(-Gemisch), zweckmässigerweise etwa 10 bis etwa 1000 Aequivalenten, vorzugsweise etwa 25 bis etwa 250 Aequivalenten.

Wird statt Methanol ein äquivalentes Gemisch aus Wasserstoff und Kohlenmonoxid und/oder Kohlendioxid als Methylierungsmittel verwendet, so entspricht die Menge des Kohlenmonoxids und/oder Kohlendioxids ebenfalls mindestens einem Aequivalent der (geschätzten) methylierbaren Stellen in dem Nicht-α-Tocopherol(-Gemisch). Auch in diesem Fall beträgt die Menge zweckmässigerweise etwa 10 bis etwa 1000 Aequivalente, vorzugsweise etwa 25 bis etwa 250 Aequivalente.

Die Geschwindigkeit, mit welcher das eventuell verdünnte Nicht-α-Tocopherol, das Nicht-α-Tocopherol-Gemisch oder das zumindest ein Nicht-α-Tocopherol enthaltende Rohmaterial über den Mischoxid-Katalysator geleitet wird, und auch die entsprechende Fliessgeschwindigkeit des Methylierungsmittels können so eingestellt und aufeinander, auf die Katalysatormenge, auf die Katalysatoraktivität, auf die Reaktionstemperatur sowie auf den Reaktionsdruck abgestimmt werden, dass die Methylierung möglichst effizient in bezug auf Umsatz und Reaktionsdauer abläuft. Dabei handelt es sich um Kontaktzeiten, die optimal im Bereich von etwa 1 bis etwa 100 Minuten liegen.

Das erfindungsgemässe Verfahren wird - wie bereits mehrmals erwähnt - unter in bezug auf das Methanol oder das äquivalente Methylierungsmittel nahe- oder überkritischen Bedingungen durchgeführt. Der kritische Druck selber beträgt etwa 77,5 bar (etwa 7,75 MPa) und die kritische Reaktionstemperatur etwa 240°C. Die Permethylierung wird bei Drücken zwischen etwa 50 bar und etwa 120 bar (etwa 5 bis etwa 12 MPa), vorzugsweise bei Drücken zwischen etwa 70 bar und etwa 90 bar (etwa 7 bis etwa 9 MPa), besonders bevorzugt bei etwa 80-85 bar (8-8,5 MPa) durchgeführt (bei Drücken unter etwa 77,5 bar, d.h. im "nahekritischen Bereich", erfolgt zwar eine schnellere Umsetzung, aber im Rohprodukt enthaltene, schwarze Verunreinigungen werden nicht aus dem Reaktor ausgetragen und können die Wirkung des Katalysators beeinträchtigen). Zudem wird beispielsweise bei etwa 50 bar (5 MPa) eine besonders hohe. Reaktionsgeschwindigkeit erreicht, allerdings zuungunsten der Katalysatorstabilität. Drücke von mehr als etwa 120 bar (12 MPa) verringern die Reaktionsgeschwindigkeit und machen die Verwendung von teuren Apparaturen erforderlich, ohne dass irgendwelche ausgleichenden Vorteile gebracht werden.

Die Reaktionstemperatur liegt im Bereich von etwa 240°C bis etwa 350°C, vorzugsweise im Bereich von etwa 280°C bis etwa 320°C.

Ein Vorteil des erfindungsgemässen Verfahrens besteht darin, dass keine erkennbare Racemisierung der optisch aktiven Zentren der zu permethylierenden Nicht-α-Tocopherole auftritt. Unter Verwendung eines Tocopherolgemisches aus natürlichen Quellen als Edukt erhält man als Produkt typischerweise RRR-α-Tocopherol mit einer optischen Reinheit von mindestens 99,5%. Auch tritt bei der Permethylierung keine nennenswerte sonstige Zersetzung des Edukts oder des Produkts auf.

Ein weiterer Vorteil besteht darin, dass der Katalysator vielmals verwendbar ist, so dass beispielsweise bei unerwünscht geringer Methylierung das erhaltene Produkt erneut unter Verwendung desselben Katalysators umgesetzt werden kann, ohne dass die Wirkung des wiederholt verwendeten Katalysators merkbar verringert ist. In diesem Zusammenhang kann an einem Katalysator überhaupt eine grosse Menge an Nicht-α-Tocopherol(en) methyliert werden, ohne dass eine merkbare Desaktivierung des Katalysators stattfindet.

Die Einfachheit der Verfahrenswerte und der Aufarbeitung sowie die hohe erreichbare Selektivität lassen das erfindungsgemässe Verfahren besonders geeignet für die grossbetriebliche Herstellung von α-Tocopherol erscheinen.

Die vorliegende Erfindung wird durch die nachfolgenden Beispiele veranschaulicht:

### Beispiel 1

### Herstellung eines typischen erfindungsgemäss verwendeten Katalysators, und typischer Reaktor (Labormassstab)

### Herstellung:

8 g (20 mmol) Aluminiumnitrat, 4 g (10 mmol) Eisen(III)nitrat, 14 g (60 mmol) Kupfer(II)nitrat und 15 g (60 mmol) Magnesiumnitrat (alle als Hydrate, d.h. als Nitrat •9H₂O, •9H₂O, •3H₂O resp. •6H₂O) wurden in 240 ml Wasser gelöst. Die resultierende Lösung wurde bei 90°C während 30 Minuten in eine Lösung von 30 g (360 mmol) Natriumhydrogencarbonat in 240 ml Wasser eingerührt. Das Gemisch wurde noch 2 Stunden bei 90°C gerührt. Der Niederschlag wurde abfiltriert und mit Wasser gewaschen, bis das Filtrat neutral reagierte. Dieser breiartige Niederschlag wurde entweder direkt bei etwa 120°C getrocknet, oder zuvor mittels einer Spritze von Hand zu "Strangpresslingen" mit einem Durchmesser von etwa 1,5 mm verformt. Der getrocknete Katalysatorvorläufer (als Brocken oder als "Strangpresslinge") wurde unter Luftzutritt etwa 4 Stunden bei 250-400°C kalciniert. Die Brocken wurden in einem Mörser verrieben, die "Strangpresslinge" in etwa 2-5 mm lange Stücke gebrochen. Der so hergestellte Katalysator wurde in einen Reaktor eingefüllt.

### Reaktor:

Der Reaktor bestand aus einem senkrecht gestellten Hochdruckrohr, das über einen Heizmantel mit Oel durch einen Thermostaten beheizt wurde. Das Rohr wies einen Innendurchmesser von 7,8 mm, eine beheizte Länge von 25 cm und eine Gesamtlänge von etwa 40 cm auf.

Im Reaktor befand sich über die gesamte Länge ein einseitig verschlossenes Rohr mit Aussendurchmesser 3,2 mm als Hülse für ein Thermoelement, mit dem die Temperatur in der Längsachse des gesamten Reaktors gemessen werden konnte.

Am unteren Ende des Reaktors befand sich die Austrittsöffnung, die mit einem Filter abgedeckt war. Der Raum zwischen Filter und dem Beginn der Heizzone wurde mit Seesand gefüllt. Darüber wurde der Katalysator eingefüllt, der je nach Menge und Schüttdichte unterschiedliche Katalysatorbetthöhen ergab. Der Raum über dem Katalysatorbett war leer.

Am oberem Ende des Reaktors befanden sich der Druckmesser, eine Berstscheibe und der Einlass für die Edukte. Zwei Hochdruckpumpen förderten Methanol und das zusätzliche Lösungsmittel (z.B. Toluen, Hexan oder weiteres Methanol) in den Reaktor. Ein Flüssigkeitsstrom - meistens der grössere - wurde vorgeheizt. Dem unbeheizten Strom wurde das Nicht-α-Tocopherol als Edukt beigemischt, wobei von reinem Lösungsmittel auf Eduktlösung umgestellt werden konnte.

Die Austrittsöffnung war mit einem Druckhalteventil verbunden, mit dem der Druck im Reaktor eingestellt werden konnte. Von dort wurde die Produktlösung in ein Auffanggefäss geleitet.

### Beispiel 2

Aus einer Lösung der Nitrate von Eisen(III) (10 mmol), Aluminium (20 mmol), Magnesium (60 mmol) und Kupfer(II) (60 mmol) wurde ein bei 350°C kalciniertes Katalysatorpulver hergestellt, das noch etwa 0,5% Natrium enthielt. 1,8 g (3,5 ml) dieses Katalysators wurden in den Reaktor gefüllt. Bei 320°C wurden mit der ersten Pumpe 1,25 ml/Min. vorgeheiztes Hexan und mit der zweiten 0,625 ml/Min. Methanol in den Reaktor gepumpt. Der Druck wurde auf 100 bar (10 MPa) eingestellt.

Dann wurden mit der zweiten Pumpe statt Methanol 3 ml einer methanolischen Lösung, die 1 g RRR-γ-Tocopherol enthielt, eingepumpt. Anschliessend wurde während 40 Minuten wieder Methanol eingepumpt, um das Produkt aus dem Reaktor zu spülen. Die gesamte Produktlösung wurde im Auffanggefäss gesammelt, eingedampft und mit Gaschromatographie (GC) analysiert. Der Eindampfrückständ enthielt 86,0 GC-Flächen-% α- und 3,9 GC-Flächen-% γ-Tocopherol. Die weitere Untersuchung ergab, dass die optische Aktivität des Eduktes erhalten war, und dass höchstens 1 Gewichts-% 2S-Isomere entstanden waren.

In einem weiteren Versuch an der gleichen Katalysatorpackung unter den gleichen Bedingungen (Temperatur, Druck und Fliessgeschwindigkeiten) wie oben wurden 3 ml einer methanolischen Lösung, die 1 g RRR-δ-Tocopherol enthielt, eingepumpt. Die eingedampfte Produktlösung enthielt 53,85 GC-Flächen-% α- und 21,94 GC-Flächen-% β/γ-Tocopherol.

### Beispiel 3

Aus einer Lösung der Nitrate von Eisen(III) (10 mmol), Aluminium (20 mmol), Magnesium (60 mmol) und Kupfer(II) (60 mmol) wurden bei 350°C kalcinierte "Strangpresslinge" als Katalysator hergestellt.

3,0 g (6 ml) dieses Katalysators wurden in den Reaktor gefüllt. Bei 300°C wurden mit der ersten Pumpe 2,25 ml/Min. Hexan und mit der zweiten 0,75 ml/Min. vorgeheiztes Methanol/Wasser (9/1 v/v) in den Reaktor gepumpt. Der Druck wurde auf 90 bar (9 MPa) eingestellt. Dann wurden mit der ersten Pumpe statt Hexan 9 ml einer Lösung, die 1 g RRR-δ-Tocopherol in Hexan enthielt, eingepumpt. Anschliessend wurde während etwa 60 Minuten wieder Hexan eingepumpt, um das Produkt aus dem Reaktor zu spülen. Die gesamte Produktlösung wurde im Auffanggefäss gesammelt, eingedampft und mit GC analysiert.

Der Eindampfrückstand wurde erneut mit Hexan auf 9 ml aufgefüllt und wie oben durch den Reaktor gepumpt und analysiert.

Auf diese Weise wurde das Tocopherol insgesamt elfmal durch den Reaktor gepumpt. Die Analysenergebnisse der einzelnen Methylierungsschritte sind in der nachfolgenden Tabelle 1 zusammengefasst:

**Tabelle 1**

| Methylierungsdurchgang | α-Tocopherol GC-Flächen-% | γ-Tocopherol GC-Flächen-% | β-Tocopherol GC-Flächen-% | δ-Tocopherol GC-Flächen-% |
|---|---|---|---|---|
| 1 | 23,7% | 4,6% | 46,5% | 19,6% |
| 2 | 43,3% | 2,3% | 44,8% | 4,4% |
| 3 | 56,9% | 1,3% | 36,6% | 1,2% |
| 4 | 65,0% | 0,8% | 29,1% | 0,4% |
| 5 | 71,4% | 0,6% | 22,0% | * |
| 6 | 73,8% | 0,7% | 19,1% | * |
| 7 | 72,5% | 0,9% | 19,9% | * |
| 8 | 79,5% | 0,9% | 12,7% | * |
| 9 | 82,4% | 0,8% | 9,8% | * |
| 10 | 85,2% | 0,8% | 7,3% | * |
| 11 | 85,9% | 0,9% | 6,1% | * |

| | | | | |
|---|---|---|---|---|
| *: nicht mehr nachweisbar | | | | |

### Beispiel 4

Aus einer Lösung der Nitrate von Eisen(III) (10 mmol), Aluminium (20 mmol), Magnesium (90 mmol) und Kupfer(II) (30 mmol) wurden bei 350°C kalcinierte "Strangpresslinge" als Katalysator hergestellt.

3,0 g (7 ml) dieses Katalysators wurden in den Reaktor gefüllt. Bei 300°C wurden mit der ersten Pumpe 3 ml/Min. Hexan und mit der zweiten 0,75 ml/Min. vorgeheiztes Methanol/Wasser (8/2 v/v) in den Reaktor gepumpt. Der Druck wurde auf 90 bar (9 MPa) eingestellt.

Dann wurden mit der ersten Pumpe statt Hexan 9 ml einer Lösung, die 1 g RRR-δ-Tocopherol in Hexan enthielt, eingepumpt. Anschliessend wurde während etwa 60 Minuten wieder Hexan eingepumpt, um das Produkt aus dem Reaktor zu spülen. Die gesamte Produktlösung wurde im Auffanggefäss gesammelt, eingedampft und mit GC analysiert.

Der Eindampfrückstand wurde erneut mit Hexan auf 9 ml aufgefüllt und wie oben durch den Reaktor gepumpt und analysiert.

Auf diese Weise wurde das Tocopherol insgesamt sechsmal durch den Reaktor gepumpt. Die Analysenergebnisse der einzelnen Methylierungsschritte sind in der nachfolgenden Tabelle 2 zusammengefasst:

**Tabelle 2**

| Methylierungsdurchgang | α-Tocopherol GC-Flächen-% | γ-Tocopherol GC-Flächen-% | β-Tocopherol GC-Flächen-% | δ-Tocopherol GC-Flächen-% |
|---|---|---|---|---|
| 1 | 27,0% | 4,1% | 39,2% | 26,7% |
| 2 | 57,4% | 1,5% | 33,5% | 3,3% |
| 3 | 71,7% | 1,2% | 21,1% | 0,5% |
| 4 | 77,3% | 1,3% | 15,2% | * |
| 5 | 80,9% | 1,5% | 11,6% | * |
| 6 | 83,7% | 1,7% | 7,9% | * |

| | | | | |
|---|---|---|---|---|
| *: nicht mehr nachweisbar | | | | |

### Beispiel 5

Aus einer Lösung der Nitrate von Eisen(III) (10 mmol), Aluminium (20 mmol), Magnesium (60 mmol) und Kupfer(II) (60 mmol) wurde ein bei 400°C kalciniertes Katalysatorpulver hergestellt.

3,95 g (9 ml) dieses Katalysators wurden in den Reaktor gefüllt. Bei 320°C wurden mit der ersten Pumpe 9 ml/Min. vorgeheiztes Hexan und mit der zweiten 2,8 ml/Min. Methanol in den Reaktor gepumpt. Der Druck wurde auf 90 bar (9 MPa) eingestellt.

Dann wurden mit der zweiten Pumpe statt Methanol 10 ml einer methanolischen Lösung, die 2,5 g eines nur etwa zur Hälfte aus Tocopherolen bestehenden Nicht-α-Tocopherolkonzentrats (Edukt) enthielt, eingepumpt. Anschliessend wurde während etwa 60 Minuten wieder Methanol mit der zweiten Pumpe eingepumpt, um das Produkt aus dem Reaktor zu spülen. Die gesammelte Produktlösung wurde eingedampft und mit GC analysiert ( Produkt 1).

In einem weiteren Versuch (wie oben) wurde mit der zweiten Pumpe statt Methanol 10 ml einer methanolischen Lösung, die 1 g des oben beschriebenen Nicht-α-Tocopherolkonzentrats (Edukt) enthielt, eingepumpt. Anschliessend wurde während etwa 60 Minuten wieder Methanol mit der zweiten Pumpe eingepumpt, um das Produkt aus dem Reaktor zu spülen. Die gesammelte Produktlösung wurde eingedampft und mit GC analysiert (Produkt 2). Die Ergebnisse sind in der nachfolgenden Tabelle 3 zusammengefasst:

**Tabelle 3**

| | α-Tocopherol | γ-Tocopherol | δ-Tocopherol | β-Tocopherol | Sterole | Rest |
|---|---|---|---|---|---|---|
| Edukt (Gew.-%) | 3% | 27% | 13% | * | 5,6% | 50% |
| Produkt 1 (GC-F1.-%) | 25,8% | 17,5% | 4,6% | 7,3% | ** | ** |
| Produkt 2 (GC-F1.-%) | 39,4% | 8,7% | 1,2% | 6,3% | ** | ** |

| | | | | | | |
|---|---|---|---|---|---|---|
| * : nicht nachweisbar | | | | | | |
| ** : nicht gemessen | | | | | | |

In einem anderen Versuch an diesem Katalysator wurden bei 320°C mit der ersten Pumpe 5 ml/Min. vorgeheiztes Hexan und mit der zweiten 2 ml/Min. Methanol in den Reaktor gepumpt. Der Druck wurde auf 90 bar (9 MPa) eingestellt.

Dann wurden mit der zweiten Pumpe statt Methanol 100 ml einer methanolischen Lösung, die 1 g δ-Tocopherol enthielt, eingepumpt. Die Produktlösung von jeweils 6,5 Minuten ( entsprechend 12,5 ml Eduktlösung) wurde getrennt gesammelt, eingedampft und mit GC analysiert. Die Ergebnisse sind in der nachfolgenden Tabelle 4 zusammengefasst:

**Tabelle 4**

| Probe (GC-Flächen-%) | α-Tocopherol | γ-Tocopherol | δ-Tocopherol | β-Tocopherol |
|---|---|---|---|---|
| 1 | 94,8% | 3,4% | 0% | 0,5% |
| 2 | 92,8% | 3,8% | 0% | 0,4% |
| 3 | 91,7% | 3,7% | 0% | 0,6% |
| 4 | 91,8% | 3,6% | 0% | 0,8% |
| 5 | 91,1% | 3,4% | 0% | 0,9% |
| 6 | 91,5% | 3,4% | 0% | 1,0% |
| 7 | 90,7% | 3,5% | 0% | 1,1% |

### Beispiel 6

Aus einer Lösung der Nitrate von Eisen(III) (10 mmol), Aluminium (20 mmol), Magnesium (60 mmol) und Kupfer(II) (60 mmol) wurden bei 350°C kalcinierte "Strangpresslinge" als Katalysator hergestellt.

3,0 g (7 ml) dieses Katalysators wurden in den Reaktor gefüllt. Bei 300°C wurden mit der ersten Pumpe 1 ml/Min. Hexan und mit der zweiten 3 ml/Min. vorgeheiztes Methanol/Wasser (9/1 v/v) in den Reaktor gepumpt. Der Druck wurde auf 85 bar (8,5 MPa) eingestellt.

Dann wurden mit der ersten Pumpe statt Hexan 3 ml einer Lösung, die 0,5 g RRR-δ-Tocopherol (Edukt) in Hexan enthielt, eingepumpt. Anschliessend wurde während etwa 60 Minuten wieder Hexan eingepumpt, um das Produkt aus dem Reaktor zu spülen. Die gesamte Produktlösung wurde im Auffanggefäss gesammelt, eingedampft und mit GC (Flächen-%-Methode) analysiert.

Der Eindampfrückstand wurde erneut mit Hexan auf 3 ml aufgefüllt und wie oben durch den Reaktor gepumpt und analysiert.

Auf diese Weise wurde das Tocopherol insgesamt elfmal durch den Reaktor gepumpt. Nach dem letzten Duchgang war noch 0,4 g Produkt vorhanden, da der Reaktionsmasse elf Analysenproben zu je 5-10 mg entnommen werden mussten. Die Analysenergebnisse der einzelnen Methylierungsschritte sind in der nachfolgenden Tabelle 5 zusammengefasst:

**Tabelle 5**

| Methylierungsdurchgang | α-Tocopherol | γ-Tocopherol | β-Tocopherol | δ-Tocopherol |
|---|---|---|---|---|
| Edukt | 0,8% | 4,5% | 0,1% | 94,0% |
| 1 | 30,7% | 3,8% | 48,2% | 11,1% |
| 2 | 57,3% | 1,3% | 34,2% | 1,2% |
| 3 | 72,8% | 0,7% | 20,3% | 0,3% |
| 4 | 81,7% | 0,6% | 11,7% | * |
| 5 | 86,4% | 0,7% | 6,9% | * |
| 6 | 89,3% | 0,6% | 3,9% | * |
| 7 | 90,8% | 0,6% | 2,2% | * |
| 8 | 91,2% | 0,7% | 1,4% | * |
| 9 | 91,6% | 0,7% | 0,8% | * |
| 10 | 91,3% | 0,8% | 0,5% | * |
| 11 | 91,8% | 0,8% | 0,3% | * |

| | | | | |
|---|---|---|---|---|
| * : nicht mehr nachweisbar | | | | |

In einem weiteren Versuch an diesem Katalysator wurden bei 320°C mit der ersten Pumpe 2,5 ml/Min. vorgeheiztes Methanol/Toluen-Gemisch (4/1 v/v) und mit der zweiten 0,5 ml/Min. Toluen in den Reaktor gepumpt. Der Druck wurde auf 85 bar (8,5 MPa) eingestellt.

Dann wurden mit der zweiten Pumpe statt Toluen 210 ml einer Lösung, die 7,089 g eines Nicht-α-Tocopherol-Konzentrates in Toluen enthielt, eingepumpt. Nachdem diese Eduktlösung eingepumpt war, wurde wieder für etwa eine Stunde auf Toluen umgestellt, um das Produkt vollständig aus dem Reaktor zu spülen. Die gesammelte Produktlösung wurde eingedampft.

Edukt und Produkt wurden genau gewogen und analysiert. Die nachfolgende Zusammenstellung (Tabelle 6) belegt, dass praktisch alles Tocopherol in RRR-α-Tocopherol umgewandelt wurde, ohne dass nennenswerte Zersetzung aufgetreten ist.

**Tabelle 6**

| Ansatz und Ausbeuteberechnung des quantitativen Versuchs | | | | | |
|---|---|---|---|---|---|
| Edukt | α-Tocopherol | γ-Tocopherol | β-Tocopherol | δ-Tocopherol | Rest |
| Molgewicht | 430,720 | 416,693 | 416,693 | 402,666 | |
| | | | | | |
| Zusammensetzung (Gewichts-%) | 3,67% | 48,51% | 1,28% | 31,30% | 15,25% |
| | | | | | |
| Einsatz-Gewicht (Total = 7,089 g) | 0,260 g (0,603 mmol) | 3,439 g (8,252 mmol) | 0,091 g (0,218 mmol) | 2,218 g (5,509 mmol) | 1,081 g |
| | | | | | |
| Theoretische Ausbeute an α-Tocopherol nach Methylierung | 0,260 g | 3,554 g | 0,094 g | 2,373 g | |
| | | | | | |
| Summe α-Tocopherol nach Theorie: | 6,281 g | | | | |

| Produkt Ausbeuteberechnung | | | | | |
|---|---|---|---|---|---|
| Gefundene Ausbeute = 7,136 g | | | | | |
| | | | | | |
| Tocopherol-Gehalt (Gewichts-%) | 83,66% | 0,81% | 1,08% | | |
| | | | | | |
| Tocopherol-Gewicht | 5,970g | | | | |
| | | | | | |
| Chemische Ausbeute (% der Theorie) | 95,05% | 0,95% | 1,23% | | |
| Analyse der Methylether auf optische Aktivität | RRR-α 93,41% | 2S-α 0,44% | RRR-β 1,24% | | |

Eine weitere Packung obigen Katalysators von 3 g (6 ml) wurde in den Reaktor gefüllt. Bei 270°C wurden mit der ersten Pumpe 1 ml/Min. Toluen und mit der zweiten 2 ml/Min. vorgeheiztes Methanol in den Reaktor gepumpt. Der Druck wurde auf 85 bar (8,5 MPa) eingestellt.

Dann wurden mit der ersten Pumpe statt Toluen nacheinander 5 Lösungen zu je 30 ml, die 0,5 bis 8 g (siehe Tabelle) Nicht-α-Tocopherolkonzentrat in Toluen enthielten, eingepumpt. Zwischen den Tocopherollösungen wurde während etwa 30 Minuten wieder Toluen eingepumpt, um das Produkt aus dem Reaktor zu spülen. Am Ende der Einpumpzeit der einzelnen Tocopherollösungen wurde eine Produktprobe genommen und mit GC (Flächen-%-Methode) analysiert. Die in der nachfolgenden Tabelle 7 aufgelisteten Resultate zeigen einen deutlichen Umsatz zu α-Tocopherol an, der von der Belastung des Reaktors, und damit von der Verweilzeit, abhängt:

**Tabelle 7**

| Probe | Eduktmenge (g)/30ml | α-Tocopherol | γ-Tocopherol | β-Tocopherol | δ-Tocopherol |
|---|---|---|---|---|---|
| Edukt | | 4,17% | 56,03% | 1,31% | 36,17% |
| 1 | 8,01 | 6,68% | 51,47% | 2,59% | 33,19% |
| 2 | 4,02 | 8,29% | 50,71% | 3,72% | 31,99% |
| 3 | 2,02 | 11,38% | 48,47% | 6,04% | 29,08% |
| 4 | 1,09 | 18,49% | 42,41% | 10,17% | 23,15% |
| 5 | 0,51 | 28,46% | 34,89% | 13,89% | 17,05% |

In einer weiteren Versuchsserie wurden bei 300°C mit der ersten Pumpe 1 ml/Min. Toluen und mit der zweiten 2 ml/Min. vorgeheiztes Methanol in den Reaktor gepumpt. Der Druck wurde wieder auf 85 bar (8,5 MPa) eingestellt.

Dann wurden mit der ersten Pumpe statt Toluen nacheinander wieder 5 Lösungen zu je 30 ml, die 0,5 bis 8 g (siehe Tabelle) Nicht-α-Tocopherolkonzentrat in Toluen enthielten, eingepumpt. Zwischen den Tocopherollösungen wurde während etwa 30 Minuten wieder Toluen eingepumpt, um das Produkt aus dem Reaktor zu spülen. Am Ende der Einpumpzeit der einzelnen Tocopherollösungen wurde eine Produktprobe genommen und mit GC (Flächen-%-Methode) analysiert. Die in der nachfolgenden Tabelle 8 aufgelisteten Resultate zeigen einen wieder deutlichen Umsatz zu α-Tocopherol an, der von der Belastung des Reaktors, und damit von der Verweilzeit, abhängt. Auch zeigt diese Serie im Vergleich zur obigen Serie, dass bei höherer Temperatur ein stärkerer Umsatz bei sonst gleichen Bedingungen erfolgt.

**Tabelle 8**

| Probe | Eduktmenge (g) / 30ml | α-Tocopherol | γ-Tocopherol | β-Tocopherol | δ-Tocopherol |
|---|---|---|---|---|---|
| Edukt | | 4,17% | 56,03% | 1,31% | 36,17% |
| 1 | 8,02 | 16,51% | 44,22% | 8,58% | 25,03% |
| 2 | 4,00 | 27,99% | 35,25% | 14,20% | 16,60% |
| 3 | 2,00 | 49,66% | 19,82% | 18,39% | 6,24% |
| 4 | 1,02 | 58,07% | 6,62% | 11,65% | 1,46% |
| 5 | 0,52 | 80,71% | 4,60% | 6,71% | 0,46% |

## Patentansprüche

1. Verfahren zur Umwandlung von Nicht-α-Tocopherolen in α-Tocopherol durch katalysierte Permethylierung mindestens eines Nicht-α-Tocopherols mit einem Methylierungsmittel, **dadurch gekennzeichnet, dass** man als Methylierungsmittel im nahe- oder überkritischen Druck- und Temperaturbereich befindliches Methanol oder ein aus Wasserstoff und Kohlenmonoxid und/oder Kohlendioxid bestehendes, dem Methanol äquivalentes Gemisch, und als Katalysator einen aus Hydrotalciten hergestellten Mischoxid-Katalysator, der zumindest Kupfer- und Magnesiumoxid sowie mindestens ein Oxid eines dreiwertigen Metalls aufweist, verwendet, und die Permethylierung bei einem Druck zwischen etwa 50 bar und etwa 120 bar (etwa 5 bis etwa 12 MPa) und bei einer Reaktionstemperatur im Bereich von etwa 240°C bis etwa 350°C durchführt.

2. Verfahren nach Anspruch 1, **dadurch gekennzeichnet, dass** man als Oxid(e) eines dreiwertigen Metalls Aluminiumoxid, Eisen(III)oxid, Vanadiumoxid, Chromoxid und/oder Galliumoxid, vorzugsweise Aluminiumoxid und/oder Eisen(III)oxid, verwendet.

3. Verfahren nach Anspruch 1 oder 2, **dadurch gekennzeichnet, dass** im Mischoxid-Katalysator das atomare Verhältnis der (gesamten) zweiwertigen Metalle zu dem bzw. den gesamten dreiwertigen Metall(en) etwa 2:1 bis etwa 10:1, vorzugsweise etwa 3:1 bis etwa 4:1, beträgt, und das atomare Verhältnis von Kupfer zu Magnesium bzw. von Kupfer zu Magnesium und weiteren vorhandenen zweiwertigen Metallen etwa 5:95 bis etwa 60:40, vorzugsweise etwa 25:75 bis etwa 50:50, beträgt.

4. Verfahren nach einem der Ansprüche 1 bis 3, **dadurch gekennzeichnet, dass** im Mischoxid-Katalysator Aluminiumoxid, Eisenoxid und gegebenenfalls Oxide weiterer dreiwertiger Metalle vorhanden sind und das atomare Verhältnis zwischen Aluminium und Eisen oder zwischen Aluminium und der Gesamtheit der weiteren dreiwertigen Metalle etwa 2:1 bis etwa 1:2, ganz bevorzugt etwa 2:1, beträgt.

5. Verfahren nach einem der Ansprüche 1 bis 4, **dadurch gekennzeichnet, dass** die in α-Tocopherol umzuwandelnden, eventuell in einem Rohmaterial enthaltenen Nicht-α-Tocopherole in einem inerten Lösungsmittel gelöst sind, wobei das inerte Lösungsmittel ein C₅₋₁₀-Alkan, z.B. Pentan, Hexan oder Heptan, oder ein Gemisch von C₅₋₁₀-Alkan, z.B. Petrolether mit Siedebereich zwischen etwa 40°C und etwa 120°C; ein cyclisches Alkan, z.B. Cyclohexan; oder ein aromatischer Kohlenwasserstoff, z.B. Toluen, ist.

6. Verfahren nach einem der Ansprüche 1 bis 5, **dadurch gekennzeichnet, dass** die Menge des als Methylierungsmittel verwendeten Methanols bzw. die Menge des Kohlenmonoxids und/oder Kohlendioxids in einem dem Methanol äquivalenten Gemisch aus Wasserstoff und Kohlenmonoxid und/oder Kohlendioxid gegenüber dem oder den Nicht-α-Tocopherol(en) etwa 10 bis etwa 1000 Aequivalenten, vorzugsweise etwa 25 bis etwa 250 Aequivalenten, der methylierbaren Stellen in dem Nicht-α-Tocopherol(-Gemisch) entspricht.

7. Verfahren nach einem der Ansprüche 1 bis 6, **dadurch gekennzeichnet, dass** die Permethylierung bei einem Druck zwischen etwa 70 bar und etwa 90 bar (etwa 7 bis etwa 9 MPa), vorzugsweise bei etwa 80-85 bar (8-8,5 MPa), und bei einer Reaktionstemperatur im Bereich von etwa 280°C bis etwa 320°C, durchgeführt wird.

## Claims

1. A process for the conversion of non-α-tocopherols into α-tocopherol by the catalytic permethylation of at least one non-α-tocopherol with a methylating agent, which process comprises using as the methylating agent in the near or supercritical pressure and temperature region methanol or a mixture which is equivalent to methanol and which consists of hydrogen and carbon monoxide and/or carbon dioxide, and as the catalyst a mixed oxide catalyst which is produced from hydrotalcites and which contains at least copper and magnesium oxide as well as at least one oxide of a trivalent metal, and carrying out the permethylation at a pressure between about 50 bar and about 120 bar (about 5 to about 12 MPa) and at a reaction temperature in the range of about 240°C to about 350°C

2. A process according to claim 1, wherein aluminium oxide, iron(III) oxide, vanadium oxide, chromium oxide and/or gallium oxide, preferably aluminium oxide and/or iron(III) oxide, is/are used as the oxide(s) of a trivalent metal.

3. A process according to claim 1 or claim 2, wherein in the mixed oxide catalyst the atomic ratio of the (total) divalent metals to the total trivalent metal(s) amounts to about 2:1 to about 10:1, preferably about 3:1 to about 4:1, and the atomic ratio of copper to magnesium or of copper to magnesium and further divalent metals present amounts to about 5:95 to about 60:40, preferably about 25:75 to about 50:50.

4. A process according to any one of claims 1 to 3, wherein aluminium oxide, iron oxide and optionally oxides of other trivalent metals are present in the mixed oxide catalyst and the atomic ratio between aluminium and iron or between aluminium and the totality of the other trivalent metals amounts to about 2:1 to about 1:2, especially about 2:1.

5. A process according to any one of claims 1 to 4, wherein the non-α-tocopherols to be converted into α-tocopherol, optionally present in a raw material, are dissolved in an inert solvent, said inert solvent being a C₅₋₁₀-alkane, e.g. pentane, hexane or heptane, a C₅₋₁₀-alkane mixture, e.g. petroleum ether having a boiling range between about 40°C and about 120°C; a cyclic alkane, e.g. cyclohexane; or an aromatic hydrocarbon, e.g. toluene.

6. A process according to any one of claims 1 to 5, wherein the amount of methanol or the amount of carbon monoxide and/or carbon dioxide in a mixture, equivalent to methanol, of hydrogen and carbon monoxide and/or carbon dioxide used as the methylating agent relative to the non-α-tocopherol(s) corresponds to about 10 to about 1000 equivalents, preferably about 25 to about 250 equivalents, of the methylatable positions in the non-α-tocopherol (mixture).

7. A process according to any one of claims 1 to 6, wherein the permethylation is carried out at a pressure between about 70 bar and about 90 bar (about 7 to about 9 MPa), preferably at about 80-85 bar (8-8.5 MPa) and at a reaction temperature in the range of about 280°C to about 320°C.

## Revendications

1. Procédé pour la conversion de non-α-tocophérols en α-tocophérols par perméthylation catalysée d'au moins un non-α-tocophérol à l'aide d'un agent de méthylation, **caractérisé en ce que** l'on utilise comme agent de méthylation du méthanol se trouvant dans la plage de pression et de température presque critique ou hypercritique ou un mélange constitué d'hydrogène et de monoxyde de carbone et/ou de dioxyde de carbone, équivalent au méthanol, et comme catalyseur un catalyseur d'oxydes mixtes fabriqué à partir d'hydrotalcites qui présente au moins de l'oxyde de cuivre et de magnésium ainsi qu'au moins un oxyde d'un métal trivalent, et **en ce que** l'on réalise la perméthylation sous une pression comprise entre environ 50 bars et environ 120 bars (environ 5 à environ 12 MPa) et à une température de réaction comprise entre environ 240°C et environ 350°C.

2. Procédé selon la revendication 1, **caractérisé en ce que** l'on utilise comme oxyde(s) d'un métal trivalent l'oxyde d'aluminium, l'oxyde ferrique(III), l'oxyde de vanadium, l'oxyde de chrome et/ou l'oxyde de gallium, de préférence l'oxyde d'aluminium et/ou l'oxyde ferrique(III).

3. Procédé selon la revendication 1 ou 2, **caractérisé en ce que**, dans le catalyseur d'oxydes mixtes, le rapport atomique de (tous) les métaux bivalents au métal trivalent ou selon le cas à tous les métaux trivalents est compris entre environ 2:1 et environ 10:1, de préférence entre environ 3:1 et environ 4:1, et le rapport atomique du cuivre au magnésium ou selon le cas du cuivre au magnésium et à d'autres métaux bivalents présents est compris entre environ 5:95 et environ 60:40, de préférence entre environ 25:75 et environ 50:50.

4. Procédé selon l'une quelconque des revendications 1 à 3, **caractérisé en ce que** de l'oxyde d'aluminium, de l'oxyde de fer et éventuellement des oxydes d'autres métaux trivalents sont présents dans le catalyseur d'oxydes mixtes et que le rapport atomique de l'aluminium au fer ou de l'aluminium à l'ensemble des autres métaux trivalents est compris entre environ 2:1 et environ 1:2, et est, de manière préférée entre toutes, d'environ 2:1.

5. Procédé selon l'une quelconque des revendications 1 à 4, **caractérisé en ce que** les non-α-tocophérols convertissables en α-tocophérols, éventuellement contenus dans une matière première, sont dissous dans un solvant inerte, ledit solvant inerte étant un C₅₋₁₀-alcane, par exemple un pentane, un hexane ou un heptane, ou un mélange de C₅₋₁₀-alcane, par exemple un éther de pétrole ayant une plage d'ébullition comprise entre environ 40°C et environ 120°C ; un alcane cyclique, par exemple un cyclohexane;ou un hydrocarbure aromatique, par exemple un toluène.

6. Procédé selon l'une quelconque des revendications 1 à 5, **caractérisé en ce que** la quantité de méthanol utilisé comme agent de méthylation ou selon le cas la quantité de monoxyde de carbone et/ou de dioxyde de carbone dans un mélange équivalent au méthanol constitué d'hydrogène et de monoxyde de carbone et/ou de dioxyde de carbone correspond, par rapport au ou aux non-α-tocophérol(s), à environ 10 à 1000 équivalents, de préférence à environ 25 à 250 équivalents, des emplacements méthylables dans le (mélange de) non-α-tocophérol(s).

7. Procédé selon l'une quelconque des revendications 1 à 6, **caractérisé en ce que** l'on réalise la perméthylation sous une pression comprise entre environ 70 bars et environ 90 bars (environ 7 à environ 9 MPa), de préférence à environ 80-85 bars (8-8,5 MPa), et à une température de réaction comprise entre environ 280°C et environ 320°C.
